Europäisches Patentamt

European Patent Office

Office européen des brevets

(11) Publication number: **0 164 896**
**B1**

(12) **EUROPEAN PATENT SPECIFICATION**

(45) Date of publication of patent specification: **01.02.89**

(51) Int. Cl.⁴: **A 61 M 5/00, A 61 M 1/00**

(21) Application number: **85303288.6**

(22) Date of filing: **09.05.85**

(54) Percutaneous access device.

(30) Priority: **25.05.84 US 613931**
**25.05.84 US 613933**

(43) Date of publication of application:
**18.12.85 Bulletin 85/51**

(45) Publication of the grant of the patent:
**01.02.89 Bulletin 89/05**

(84) Designated Contracting States:
**AT BE CH DE FR GB IT LI LU NL SE**

(56) References cited:
**EP-A-0 039 183**
**EP-A-0 078 565**
**GB-A-2 056 282**
**US-A-3 783 868**

(73) Proprietor: **THERMEDICS, INC.**
**470 Wildwood Street P.O. Box 2999**
**Woburn Massachusetts (US)**

(72) Inventor: **Poirier, Victor L.**
**12, Belmont Drive**
**Chelmsford Massachusetts (US)**
Inventor: **Clay, Warren C.**
**73 Mudge Street**
**Lynn Massachusetts (US)**
Inventor: **Daly, Benedict D. T.**
**12 Wildwood Circle**
**Wellesley Massachusetts (US)**

(74) Representative: **Brunner, Michael John et al**
**GILL JENNINGS & EVERY 53-64 Chancery Lane**
**London WC2A 1HN (GB)**

Courier Press, Leamington Spa, England.

**Description**

This invention relates to devices for permitting access to the body through the skin, i.e., percutaneous access devices, and more particularly to skin penetration devices which may be used as an access port into the body for extended periods of time.

Percutaneous access devices are useful when frequent or long-term access to the body is required as in kidney dialysis, drug delivery, intravenous feeding, ostomies, and transmission of energy to intracorporeal blood pumps. practical devices for long-term skin penetration in humans, however, have not been successful prior to the present invention because skin adjacent to the implanted devices will not heal to form a tight barrier to infection. Instead, when a foreign device is implanted for percutaneous access, epidermal cells begin to migrate, each seeking to surround itself completely with other similar cells. The epidermal cells thus grow down the sides of the device in an attempt to expel it. Deep sinus tracts form and body fluids are exuded at the interface between the device and adjacent tissue, forming a bed for infection. The percutaneous device, if not expelled spontaneously, must be removed to allow the infection to be cured.

Another drawback of presently available skin penetration devices relates to the difficulty of correcting problems with tubes mounted to, and extending through, such devices. Catheter tubes which are used, for example, in continuous ambulatory peritoneal dialysis or continuous infusion of drugs may become misaligned, kinked, and blocked, or coated with fibrin. With existing technology such catheters cannot readily be removed without disturbing the percutaneous access device, and thus they must be surgically removed and replaced with another access device at a different site. Current technology does not, therefore, readily permit multiple use of an implanted percutaneous access device wherein one catheter may be substituted for another, nor does it allow catheter removal followed by plugging of the device for use at a later time.

EP-A-0039183 discloses a percutaneous access device for implantation in humans or animals comprising a main body including a substantially flat disk-shaped skirt, a neck integral with and substantially normal to the skirt, the neck and skirt being made of a biocompatible material and including a bore therethrough, and a bed of porous material extending over the surface of the skirt.

The device, however, raises the problem that if the bed of porous material is such as to produce good skin interfacing· the device will eventually become extruded. If it is such that good skin interfacing is not accomplished, a sinus tract develops around the device through which bacteria gain entrance to the underlying tissues.

The aim of the present invention is to overcome this dilemma.

To this end, according to this invention, a device as disclosed in EP-A-0039183 is characterised in that

the biocompatible material is polyurethane; and

in that the bed of porous material is formed by a first porous bed surrounding and attached to a portion of the neck adjacent the surface of the skirt and extending along a portion of the surface of the skirt adjacent the neck, the first porous bed including a multiplicity of interconnected pores of a size in the range of from 75 to 125 microns such that upon implantation of the skirt just below the dermis, the pores permit downgrowth of epidermal cells through the bed at a controlled rate, and a second porous bed forming a junction on the skirt with one end of the first bed, the second porous bed being attached to and covering a portion of the skirt not overlain by the first bed and including pores of a size in the range of from 400 to 800 microns to facilitate the infiltration of fibroblasts.

The percutaneous access device in accordance with the invention promotes formation of a tight barrier to infection by controlling the migration of epidermal cell downgrowth and providing a stable junction to ensure mechanical and biologic stability.

A key feature of the percutaneous access device in accordance with the invention is the double porous bed positioned along portions of the neck and skirt which promotes the formation of a tight infection-free barrier or biologic seal between the device and surrounding tissue. The first bed may be formed of a material such as polytetrafluoroethylene (PTFE) having pores of a specified size and having a specified path length. The first bed preferably covers a lower portion of the neck and extends along a curved transition zone between the neck and the skirt. The second, more porous bed, formed of a material such as a polyester velour, covers at least some, and desirably all, of the remainder of the skirt and forms a junction, which is critical, with the first bed. The path length of the first bed along the neck and skirt and the pore sizes of both beds are preferably selected such that, upon implantation of the skirt in subcutaneous tissue, the first bed permits a controlled rate of downward growth of epidermal cells while the second bed promotes formation of collagen and its displacement of body fluids in the large pores of the second bed.

In a preferred form of the device, the first stage has a path length of about 0.25 inches (6.4 mm). By the time epidermal downgrowth reaches the junction between the first and second beds, mature collagen has formed in the second bed, causing epidermal cell downgrowth and sinus tract penetration to cease and a tight, infection-free seal to form at or near the junction. The tight dermal/biomaterial barrier stabilizes the implanted percutaneous access device, permitting its long-term use in the body.

Two examples of devices in accordance with the invention are illustrated in the accompanying drawings in which:

Figure 1 is a top or plan view of one example;

Figure 2 is a cross-sectional view of the device of Figure 1 taken along the line 2-2;

Figure 3 is a cross-sectional view to a larger scale of one-half of the device shown in Figures 1 and 2 illustrating in schematic form the histology associated with a stabilized, implanted PAD;

Figure 4 is a cross-sectional view of a second example illustrating a tube-mounting arrangement permitting removal and replacement of tubes extending through the implanted device; and,

Figure 5 is a cross-sectional view of the second example similar to Figure 4 but showing the device plugged for further potential use.

The long-term percutaneous access device (PAD) shown and described herein overcomes the fundamental instability of known devices in that it promotes the formation of a tight, infection-free barrier. The biologic barrier or seal is formed in a predetermined area adjacent to the device by the use of a unique two-stage bed of controlled porosity and length covering the main structural body of the percutaneous access device.

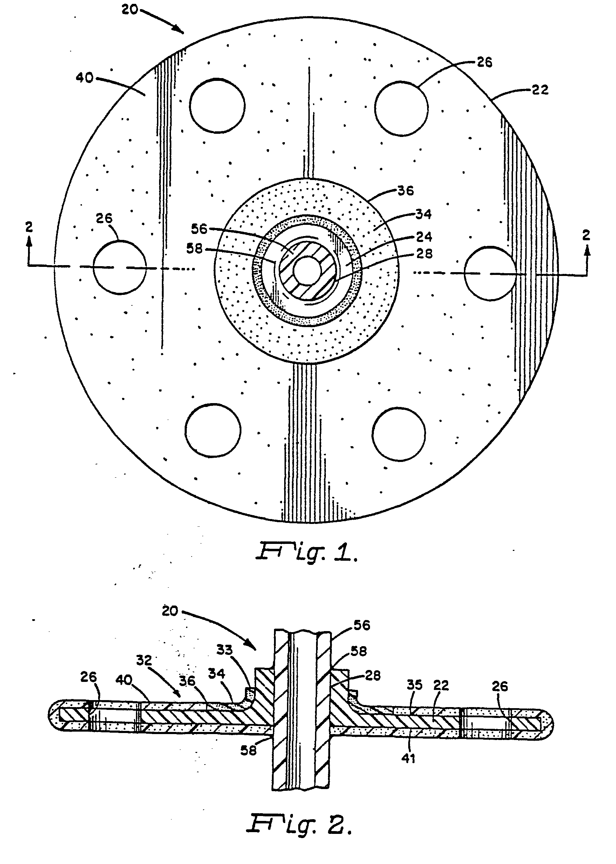

A preferred percutaneous access device 20 for implantation in humans is shown in Figures 1 and 2. The PAD 20 is buttonlike in appearance and includes a generally flat flange or skirt 22 for anchoring the device and a hub or neck 24, integral with and generally normal to the skirt 22. The skirt 22 and the neck 24 are formed of a nontoxic biocompatible semiflexible material such as a semirigid polyurethane. A suitable polyurethane is Tecoflex® EG-60D, available from Thermedics Inc. of Woburn, Massachusetts. Preferably the skirt 22 is disk-shaped and has a diameter in the range of about l.2 to l.8 inches (30.5 to 46 mm) and a thickness of about 0.010 to 0.100 inches (0.45 to 2.5 mm). One or more holes 26 are provided in the skirt 22 to encourage tissue penetration for increased anchoring of the PAD 20, to facilitate lymphatic drainage, and to reduce swelling. The neck 24 has a diameter of about 0.25 to 0.50 inches (6.3 to 12.7 mm) and flares upward from the skirt 22 a distance of about 0.08 to 0.40 inches (2.0 to 10.2 mm) so as to extend through the epidermal and dermal layers of a patient when the skirt 22 is implanted in the subcutaneous tissue (see Figure 4). A central hole or bore 28 is provided in the neck 24 and also extends through the skirt 22 to accommodate a tube 30 (Figure 4).

To promote healing of skin and the formation of a tight, infection-free barrier between the percutaneous access device 20 and adjacent tissues following implantation of the PAD 20, the skirt 22 and at least the lower portion of the neck 24 are covered by a two-stage porous bed 32. A first stage 34 of the bed 32 overlies the lower portion of the neck 24, preferably commencing at .a location such that the top 33 of this first stage 34 is positioned just below the epidermal layer when the PAD is implanted. The first stage 34 also extends along part of the upper surface 35 of the skirt 22 e.g., a distance of about 0.10 to 0.25 inches (2.5 to 6.3 mm) terminating in a junction 36 formed between adjacent ends of the first stage 34 and a second stage 40.

A preferred material for the first stage 34 of the porous bed is polytetrafluoroethylene (PTFE) having pore sizes of about 50 125 microns and a thickness of about 0.020 inches (0.5 mm). For example, the first stage of the porous bed may be formed of Impra® 15:l, a PTFE material available from Impra Inc., of Tempe, Arizona, and formed by extrusion followed by stretching to fifteen times the extruded length. The second stage 40 of the porous bed 32 covers at least the remainder of the upper surface 35 of the skirt 22 and preferably the lower surface 41 as well. This stage 40 of the porous bed 32 may be formed of a material such as a polyester velour (e.g. Dacron® velour available as part No. 600K61121 available from the United States Catheter and Instrument Company of Glenfalls, New York.) This material is a woven fabric with loose strands to allow for cell infiltration, and its pore sizes are considerably larger than those of the first stage 34 of the porous bed 32, typical values. being about 400 to 800 microns.

Both porous stages are tightly bonded to the underlying substrate (the skirt 22 and the neck 24) by a suitable adhesive such as No. 1-MP polyurethane adhesive available from Thermedics Inc. of Woburn, Massachusetts. To improve its adhesion to the skirt 22 the Dacron® velour may be chemically stripped, as bv washing it in distilled water adjusted to a ph of 10.

It is essential to the successful long-term implantation of the percutaneous access device of the invention that the path length of the first stage 34 of the porous bed 32 along the neck 24 and the skirt 22, and the pore sizes of both stages 34 and 40, be properly selected to fulfill the different functions performed by these stages. Accordingly, the material of the first stage 34 has pores of about 50-125 microns in size, preferably about 75-125 microns, a size which permits downgrowth of epidermal cells, but at a rate far less than would occur in a material having larger pores. The biomaterial of the second stage has pores of about 400 to 800 microns in size, large enough to allow penetration and viability of cells such as fibroblasts which displace body fluids from these pores and synthesize collagen. The controlled rate of epidermal cell downgrowth allowed by the first stage 34, together with a first stage length of about 0.25 inches, is sufficient to prevent epidermal cells from reaching the junction 36 between the stages 34 and 40 until mature collagen is formed in the pores of the second stage 40 (typically two to six months following implantation of the device 20). The presence of mature collagen in the second stage 40 terminates the growth of epidermal cells at or near the junction 36, thus forming a stable, tight, dermal/biomaterial barrier.

For purposes of the invention described and claimed herein, pore size is defined as the diameter of a circle whose area is equal to the area of an equivalent opening or void in the bed structures. The pores may, for example, be formed between threads or filaments of the porous bed structures, the filaments preferably being utilized in multiple layers positioned in random fashion to avoid alignment of pores in adjacent layers. The resulting structure of the porous bed stages has voids or pores which are interconnected in tortuous paths along the length of the bed, permitting controlled growth of cells through the pores and strong mechanical bonding due to wrapping of cells around the filaments. It is essential that the pores be interconnected so that cells may infiltrate and, particularly with reference to the first stage of the porous bed, so that epidermal cells may grow down through the pores of the first stage at a controlled rate.

The necessity of proper pore size selection and of a two-stage bed of porous material is indicated by the following discussion of the consequences of alternative structures. For example, were a single stage bed of small pore size material utilized in the percutaneous access device 20, such pores would not permit survival of infiltrating fibroblasts and the formation of mature collagen needed to halt the growth of epidermal cells. were a single stage bed of large pore size material utilized, body fluids would be wicked to the external environment, providing a moist environment for bacterial infection. Note that even in a two-stage bed, use in the first stage of material having pores which are too small will not sufficiently retard epidermal cell downgrowth since rapid downgrowth of epidermal cells will occur around the first stage. In each of the above-noted alternatives no stable, infection-free barrier would form to permit long-term implantation of the percutaneous access device.

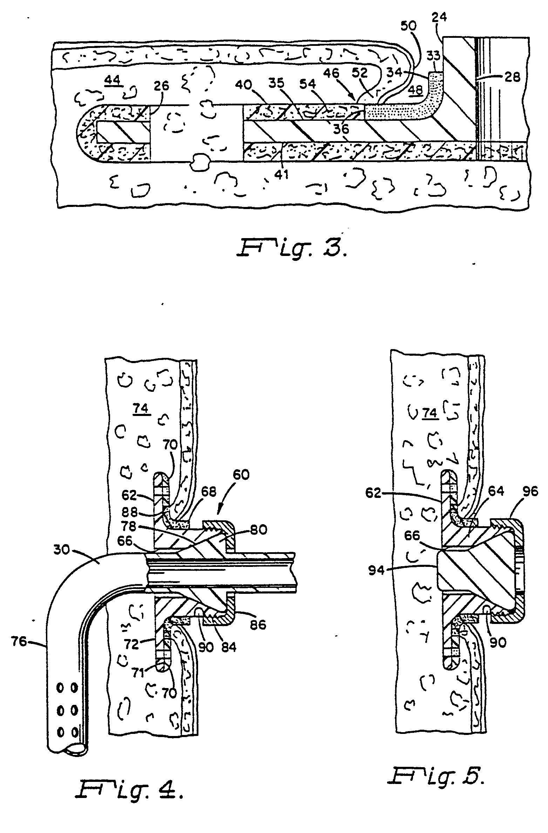

Fig. 3 shows in diagram form the skin and tissue structure adjacent to a stabilized implanted percutaneous access device 20 as disclosed herein. For ease of illustration, only one-half of the symmetrical PAD 20 is indicated and no tube is shown extending through the central bore 28. The histology illustrated, which is typical of that observed about six months after implantation of the device 20 in the subcutaneous layer 44, is characterized by a stable interface 46 between the skin and the PAD 20. The interface or barrier 46 is located near the junction 36 between the PTFE first stage 34 and the polyester velour second stage 40 of the porous bed and lies at the end of a sinus 48 which forms and progresses downward and along the porous bed 32 during the 2-6 month period of stabilization of the implanted device 20. A thin epidermal layer 50 lines the sinus 48, and the connective tissue 52 in the interface 46 is similar to dermis. Mature collagen bundles 54 are present in the polyester velour second stage 40, and the implant site is generally infection-free. The interface 46 remains essentially stable for periods of one year or longer.

Percutaneous access devices having the general configuration shown in Figs. 1-3 (but without a central bore 28 and tube) were implanted in various positions of pigs and many survived a full year before being electively explanted. Examination of the connective tissue of these devices showed structure similar to that of Fig. 3, including an interface near the junction of the two stages of the porous bed having mature dense collagen similar to dermis.

The first stage 34 of the porous bed may, instead of being formed of PTFE, be fabricated of multiple layers of filaments of a polyurethane such as Tecoflex® EG-60D, available from Thermedics Inc. of Woburn, Massachusetts (Tecoflex is a registered trademark of Thermedics for medical grade urethane elastomeric materials). This polyurethane, which is formed as the reaction product of dicyclohexyl methane diisocyanate, polytetramethylene ether glycol, and 1, 4 butane diol, has shown excellent cell attachment characteristics and biocompatibility in invitro tests of several biomaterials with human skin fibroblasts. when used in the first stage of the porous bed, the filaments preferably have a diameter of from about 0.004 to 0.015 inches and are formed into a structure having about three to ten layers. The structure is fabricated such that it has spaces or open areas between filaments of about 0.001 to 0.004 inches (as measured from electron micrographs of surfaces and cross-sections of the porous bed). One method of fabricating the first stage 34 of the porous bed is to wind in random fashion the polyurethane filaments onto a mandrel and then to form a bonded structure by heating the mandrel for example, to a temperature of about 110°C for approximately one half hour. An alternative to heating the wound filaments is to overcoat them with an adhesive such as a dilute solution of the polyurethane Tecoflex EG-60D plus a solvent.

Tecoflex EG-60D polyurethane may also be used in the second stage of the porous bed in place of Dacron velour. A suitable loose mesh structure includes about 3 to 10 layers of filaments of 0.004 to 0.015 inch diameter formed with interfilament spacings of about 0.015 to 0.030 inches. Also, the filaments may be reinforced with a higher modulus core such as Dacron.

As suggested in Fig. 2, the percutaneous access device 20 can be assembled prior to implant with an appropriate catheter 56. A permanent seal or bond 58 can be accomplished by using an adhesive such as 1-MP polyurethane adhesive available from Thermedics, Inc. of Woburn, Massachusetts.

Figs. 4 and 5 illustrate a percutaneous access device which facilitates replacement and removal of tubes in a PAD without the need for surgery and with minimal interference with an implanted PAD. Non-surgical removal of catheters may be particularly desirable for replacement of kinked, blocked, or misaligned catheters or for multiple use of a single, permanently-implanted PAD. The percutaneous access device 60 shown in Fig. 4, as in the earlier-described PAD, has a buttonlike main body including a skirt 62 and a neck 64 with a bore 66 therethrough. Portions of the neck 64 and the skirt 62 are covered by a porous bed which preferably comprises a first stage 68 and an adjoining second stage 70. The second stage 70 of the porous bed may cover just the upper surface 71 of the skirt 62 as shown in Figs. 4 and 5 or it may also cover the lower surface 72 as in the device illustrated in Figs. 1-3. The device 60 as shown has its skirt 62 implanted in the subcutaneous tissue 74 of an abdominal wall of a patient with a catheter 30 mounted to and extending through the PAD and having its implanted end 76 directed generally downward.

To permit mounting of the catheter 30 to, and its removal from, the percutareous access device 60, the PA 60 includes a connector lock, a preferred type being the screw ring lock illustrated in Figs. 4 and 5, but which may instead comprise other forms of twist locks or connectors which can be operated with minimal disturbance of the implanted device 60. In conjunction with the lock shown in Fig. 4, the neck 64 of the PAD 60 includes a tapered portion 78 which accommodates a seal 80. The seal 80 may, for example, be formed of an elastomeric body which fits over the tube 30 and is shaped to fill the enlarged portion of the bore 66. The end of the neck opposite the skirt 62 also has a threaded outer surface 84 to engage a screw ring 86 which fits over the catheter 30 and, when tightened, presses the seal 80 against the neck 64 and the tube 30. A metallic insert such as titanium may be used to insure proper attachment of the screw ring or connector lock.

It is important for re-use of the percutaneous access device 60 illustrated in Figs. 4 and 5 that there be minimal disturbance to the interface between the PAD 60 and adjacent tissue during removal and replacement of the tube 30, and particularly that the infection-free barrier formed near the junction 88 between stages 68 and 70 of the porous bed not be broken. To minimize such disturbance, two or more recesses or indentations 90 may be provided in the outer circumference of the neck 64 of the PAD 60 adjacent to the threaded surface 84 so that during tightening or removal of the screw ring 86 the neck 64 may be tightly restrained against twisting by means of a spanner wrench having gripping elements receivable in the indentations 90.

Fig. 5 shows a percutaneous access device 60 which carries a plug 94 instead of a catheter, a configuration which may be desirable for patients not requiring a catheter on a continuous basis, but for whom periodic or future use of a catheter is likely. The plug 94, which is preferably formed of a biocompatible elastomeric material, may be retained by a non-perforated screw cap 96 as illustrated in Fig. 5, or the screw ring 86 shown in Fig. 4 or any other suitable holder may be employed.

It is to be understood that the forms of the percutaneous access device shown and described herein are preferred embodiments and that the device may be constructed of various other biocompatible materials and with some change in shape and size. The invention is defined as all embodiments and their equivalents within the scope of the claims which follow.

## Claims

1. A percutaneous access device for implantation in humans or animals comprising:
a main body including a substantially flat disk-shaped skirt (22, 62), a neck (24, 64) integral with and substantially normal to the skirt (22, 62), the neck and skirt being made of biocompatible material and including a bore (28, 66) therethrough to accommodate a tube (56, 76), and a bed of porous material extending over the surface of the skirt, characterized in that
the biocompatible material is polyurethane; and,
in that the bed of porous material is formed by a first porous bed (34, 68) surrounding and attached to a portion of the neck (24, 64) adjacent the surface of the skirt and extending along a portion of the surface of the skirt (22, 62) adjacent the neck (24, 64), the first porous bed (34, 68) including a multiplicity of interconnected pores of a size in the range of from 75 to 125 microns such that upon implantation of the skirt (22, 64) just below the dermis, the pores permit downgrowth of epidermal cells through the bed at a controlled rate, and a second porous bed (40, 70) forming a junction (36, 88) on the skirt (22, 62) with one end of the first bed (34, 68), the second porous bed (40, 70) being attached to and covering a portion of the skirt (22, 62) not overlain by the first bed (34, 68) and including pores of a size in the range of from 400 to 800 microns.

2. A device according to claim 1, wherein the first bed is formed of from three to ten layers of filaments of a polyurethane material, the filaments having a diameter in the range of from 0.004 to 0.015 inches (0.1 to 0.38 mm) and being spaced apart a distance of from 0.001 to 0.004 inches (0.025 to 0.1 mm).

3. A device according to claim 1 or claim 2, wherein the second bed (40) is formed of from three to ten layers of filaments of a polyurethane material, said filaments having a diameter in the range of from 0.004 to 0.015 inches (0.1 to 0.38 mm) and being spaced apart a distance of from 0.015 to 0.030 inches (0.38 to 0.76 mm).

4. A device according to any one of claims 1 to 3, wherein the first bed (34) has a path length along the neck (24) and skirt (22) of at least about 0.25 inches (6.4 mm).

5. A device according to claim 1, wherein the first bed (34) is made of polytetrafluoroethylene.

6. A device according claim 1, in which the second bed is made of a polyester.

7. A device according to any one of the preceding claims in which the second bed (40) covers substantially all of the outer surface of the skirt (22) not overlain by the first bed (34).

8. A device according to claim 4, wherein the path length of the first bed (34, 68) and the pore sizes of the beds (34, 40) are selected such that, upon implantation of the skirt (22) in subcutaneous tissue adjacent to the dermis, the first bed (34) permits a controlled rate of epidermal cell downgrowth and the second bed (40) permits the formation of mature collagen near the junction (36) between the first and second beds (34, 40) substantially prior to the time epidermal cell downgrowth reaches said junction, whereby said epidermal cell downgrowth is halted near the junction and a tight, biologic seal is formed between the device and adjoining tissue near the junction (36).

9. A device according to any one of the preceding claims further including a catheter tube (56) extending through the bore (28) and bonded to the neck (24).

10. A device according to any one of the preceding claims, further comprising lock means (86, 96) for releasably locking a workpiece (80, 94) within the bore (66) of the neck (64) such that the workpiece (80, 94) may be inserted and replaced without removal of said percutaneous access device (60) from an implanted position and without any substantial disturbance to skin and tissue (74) near said device, said lock means (86, 96) including means (90) for preventing twisting of said neck (64) during locking and release of said workpiece (80, 94).

11. A device according to claim 10, wherein the workpiece comprises a tube (76) for conveying material, and said means for releasably locking the tube comprises a flexible seal (80) shaped to fit within the bore (78) of said neck (64) and to encircle a portion of said tube, and a cap (86) having a hole therethrough to accommodate the tube, said cap (86) being attachable to the neck (64) and being adapted to compress the seal (80) against the tube (76) and neck (64).

12. A device according to claim 10, wherein the workpiece comprises a plug (94) shaped for insertion into the bore (66) of the neck (64), and the means for releasably locking the plug (94) comprises a cap (96) attachable to the neck (64) over the plug (94).

13. A device according to claim 11, wherein the cap comprises a screw ring slidable along the tube, and the neck (64) includes a screw threaded portion (84) engagable with the cap.

14. A device according to any of claims 10 to 13, wherein the means for preventing twisting comprises a pair of recesses (90) in the outer surface of the neck (64) to permit the holding of the neck (64) in position during locking and release of the workpiece (80, 94).

15. A device according to claim 11, wherein a portion of the neck (64) is shaped to form a tapered cavity (78) in the bore (66) and the flexible seal (80) comprises an elastomeric material receivable in the cavity (78).

## Patentansprüche

1. Perkutane Zugangsvorrichtung zur Implantation in Menschen oder Tieren, umfassend:
   einen Hauptkörper mit einer im wesentlichen flachen, scheibenförmigen Einfassung (22, 62), einem Hals (24, 64) integral mit und im wesentlichen senkrecht (normal) zu der Einfassung (22, 62), wobei der Hals und die Einfassung aus biokompatiblem Material hergestellt sind und eine durchgehende Bohrung (28, 66) aufweisen, um eine Röhre (56, 76) zu akkommodieren, und einer Lage aus porösem Material, die sich über die Oberfläche der Einfassung erstreckt, dadurch gekennzeichnet, daß das biokompatible Material Polyurethan ist und daß die Lage aus porösem Material gebildet ist durch eine erste poröse Lage (34, 68), die den Hals umgibt und an einem Bereich des Halses (24, 64) benachbart der Oberfläche der Einfassung befestigt ist und sich entlang eines Bereiches der Oberfläche der Einfassung (22, 62) benachbart des Halses (24, 64) erstreckt, wobei die erste poröse Lage (34, 68) eine Vielzahl untereinander verbundener Poren einer Größe im Bereich von 75 bis 125 um beinhaltet, so daß nach Implantation der Einfassung (22, 64) knapp unter der Haut, die Poren ein Tiefenwachstum epidermaler Zellen durch die Lage mit einer kontrollierten Geschwindigkeit erlauben, und eine zweite poröse Lage (40, 70), die auf der Einfassung (22, 62) mit einem Ende der ersten Lage (34, 68) eine Verbindung (36, 88) bildet, wobei die zweite poröse Lage (40, 70) an der Einfassung befestigt ist und einen Bereich der Einfassung (22, 62) bedeckt, der nicht durch die erste poröse Lage (34, 68) überlagert ist und Poren im Bereich von 400 bis 800 um beinhaltet.

2. Vorrichtung nach Anspruch 1, wobei die erste Lage aus drei bis zehn Schichten aus Filamenten eines Polyurethanmaterials gebildet ist, wobei die Filamente einen Durchmesser im Bereich von 0.004 bis 0,015 inch (0,1 bis 0,38 mm) besitzen und in einem Abstand von 0,001 bis 0,004 inch (0,025 bis 0,1 mm) auseinanderliegen.

3. Vorrichtung nach Anspruch 1 oder 2, wobei die zweite Lage (40) aus drei bis zehn Schichten aus Filamenten aus einem Polyurethanmaterial gebildet ist, wobei die Filamente einen Durchmesser im Bereich von 0,004 bis 0,015 inch (0,1 bis 0,38 mm) besitzen und in einem Abstand von 0,015 bis 0,030 inch (0,38 bis 0,76 mm) auseinanderliegen.

4. Vorrichtung nach einem der Ansprüche 1 bis 3, wobei die erste Lage (34) eine Schichtlänge entlang des Halses (24) und der Einfassung (22) von mindestens etwa 0,25 inch (6,4 mm) besitzt.

5. Vorrichtung nach Anspruch 1, wobei die erste Lage (34) aus Polytetrafluorethylen hergestellt ist.

6. Vorrichtung nach Anspruch 1, wobei die zweite Lage aus einem Polyester hergestellt ist.

7. Vorrichtung nach einem der vorangehenden Ansprüche, wobei die zweite Lage (40) im wesentlichen die gesamte Außenoberfläche der Einfassung (22), die nicht durch die erste Lage (34) überlagert ist, bedeckt.

8. Vorrichtung nach Anspruch 4, wobei die Schichtlänge der ersten Lage (34, 68) und die Porengrößen der Lagen (34, 40) so gewählt sind, daß nach Implantation der Einfassung (22) in subkutanes Gewebe benachbart der Haut, die erste Lage (34) .

eine kontrollierte Geschwindigkeit des epidermalen Zelltiefenwachstums erlaubt und die zweite Lage (40) die Ausbildung von reifem Kollagen nahe der Verbindung (36) zwischen der ersten und zweiten Lage (34, 40) im wesentlichen vor dem Zeitpunkt, zu dem das epidermale Zelltiefenwachstum diese Verbindung erreicht, erlaubt, wodurch das epidermale Zelltiefenwachstum nahe der Verbindung zum Stillstand kommt und eine dichte biologische Abdichtung zwischen der Vorrichtung und angrenzendem Gewebe nahe der Verbindung (36) gebildet wird.

9. Vorrichtung nach einem der vorangehenden AnsPrüche, umfassend weiterhin eine Katheterröhre (56), die sich durch die Bohrung (28) erstreckt und mit dem Hals (24) verbunden ist.

10. Vorrichtung nach einem der vorangehenden Ansprüche, umfassend weiterhin ein Verschlußmittel (86, 96) zur lösbaren Verschließung eines Arbeitsstückes (80, 94) innerhalb der Bohrung (66) des Halses (64), so daß das Arbeitsstück (80, 94) eingesetzt und ersetzt werden kann, ohne Entfernung der perkutanen Zugangsvorrichtung (60) aus einer implantierten Position und ohne irgendeine wesentliche Störung der Haut und des Gewebes (74) in der Nähe der Vorrichtung, wobei das Verschlußmittel (86, 96) ein Mittel (90) zur Verhinderung einer Verdrehung des Halses (64) während dem Einschließen und Ersetzen des Arbeitsstückes (80, 94) beinhaltet.

11. Vorrichtung nach Anspruch 10, wobei das Arbeitsstück eine Röhre (76) zum Transport von Material umfaßt und wobei das Mittel zum lösbaren Verschließen der Röhre eine flexible Abdichtung (80), die so geformt ist, daß sie in die Bohrung (78) des Halses (64) einpaßt und einen Teil der Röhre umschließt, und eine Kappe (86) mit einem Loch, um die Röhre zu akkommodieren, umfaßt, wobei die Kappe (86) an dem Hals (64) befestigbar ist und so ausgelegt ist, daß sie die Abdichtung (80) gegen die Röhre (76) und den Hals (64) drückt.

12. Vorrichtung nach Anspruch 10, wobei das Arbeitsstück einen Stöpsel (94), der zum Einsatz in die Bohrung (66) des Halses (64) geformt ist, umfaßt und wobei das Mittel zum lösbaren Verschließen des Stöpsels (94) eine Kappe (96) umfaßt, die am Hals (64) über dem Stöpsel (94) befestigbar ist.

13. Vorrichtung nach Anspruch 11, wobei die Kappe einen entlang der Röhre führbaren Gewindering umfaßt und der Hals (64) einen für die Kappe eingreifbaren Schraubgewindebereich (84) beinhaltet.

14. Vorrichtung nach einem der Ansprüche 10 bis 13, wobei das Mittel zur Verhinderung einer Verdrehung ein Paar Ausnehmungen (90) in der Außenfläche des Halses (64) umfaßt, um das Halten des Halses (64) in Position während dem Verschließen und Herausnehmen des Arbeitsstückes (80, 94) zu gestatten.

15. Vorrichtung nach Anspruch 11, wobei ein Bereich des Halses (64) so geformt ist, daß eine konische Vertiefung (78) in der Bohrung (66) gebildet wird und wobei die flexible Abdichtung (80) ein elastomeres Material, das in die Vertiefung (78) aufnehmbar ist, umfaßt.

**Revendications**

1. Dispositif d'accès percutané destiné à une implantation sur des humains ou des animaux, comprenant:
un corps principal comportant une jupe en forme de disque et sensiblement plate (22, 66), un collet (24, 64) d'un seul tenant avec et sensiblement perpendiculaire à la jupe (22, 66), le collet et la jupe étant réalisés en une matière biocompatible et comprenant un alésage (28, 66) les traversant pour recevoir un tube (56, 76), et un matelas en une matière poreuse s'étendant sur la surface de la jupe, caractérisé en ce que
la matière biocompatible est du polyuréthane; et
le matelas de matière poreuse est formé par un premier matelas poreux (34, 68) entourant et fixé à une partie du collet (24, 64) adjacente à la surface de la jupe et s'étendant le long d'une partie de la surface de la jupe (22, 62) qui est adjacente du collet (24, 64), le premier matelas poreux (34, 68) comprenant une multiplicité de pores interconnectés de dimensions situées dans la plage allant de 75 à 125 microns de manière qu'après implantation de la jupe (22, 64) juste au-dessous du derme, les pores permettent une croissance vers le bas de cellules épidermiques à travers le matelas à une vitesse contrôlée, et un second matelas poreux (40, 70) formant une jonction (36, 88) sur la jupe (22, 62) avec une extrémité du premier matelas (34, 68), le second matelas poreux (40, 70) étant fixé à et recouvrant une partie de la jupe (22, 62) qui n'est pas recouverte par le premier matelas (34, 68) et comprenant des pores ayant des dimensions situées dans la plage allant de 400 à 800 microns.

2. Dispositif selon la revendication 1, dans lequel le premier matelas est formé de trois à dix couches de filaments de polyuréthane, les filaments ayant un diamètre situé dans la plage allant de 0,1 à 0,38 mm (0,004 à 0,015 pouce) et étant espacés d'une distance de 0,25 à 0,1 mm (0,001 à 0,004 pouce).

3. Dispositif selon la revendication 1 ou la revendication 2, dans lequel le second matelas (40) est constitué de trois à dix couches de filaments de polyuréthane, lesdits filaments ayant un diamètre compris dans la plage allant de 0,1 à 0,38 mm (0,004 à 0,015 pouce) et étant espacés d'une distance comprise entre 0,38 et 0,76 mm (0,015 à 0,030 pouce).

4. Dispositif selon l'une quelconque des revendications 1 à 3, dans lequel le premier matelas (34) a une longueur de parcours le long du collet (24) et de la jupe (22) qui est d'au moins environ 6,4 mm (0,25 pouce).

5. Dispositif selon la revendication 1, dans lequel le premier matelas (34) est réalisé en polytétrafluoréthylène.

6. Dispositif selon la revendication 1, dans lequel le second matelas est constitué en un polyester.

7. Dispositif selon la revendication 1, dans lequel le second matelas (40) recouvre sensiblement la totalité de la surface extérieure de la jupe (22) qui n'est pas recouverte par le premier matelas (34).

8. Dispositif selon la revendication 4, dans lequel la longueur du parcours du premier matelas (34, 68) et la dimension des pores des matelas (34, 40) sont choisies de manière qu'après implantation de la jupe (22) dans un tissu subcutané adjacent au derme, le premier matelas (34) permette une croissance vers le bas de cellules épidermiques à une vitesse contrôlée et le second matelas (40) permette la formation d'un collagène mature à proximité de la jonction (36) entre les premier et second matelas (34, 40) sensiblement avant le moment où la croissance vers le bas des cellules épidermiques atteint ladite jonction, la croissance vers le bas des cellules épidermiques étant ainsi arrêtée à proximité de la jonction et un joint biologique étanche étant constitué entre le dispositif et le tissu avoisinnant à proximité de la jonction (36).

9. Dispositif selon l'une quelconque des revendications précédentes, comprenant en outre un tube de cathéter (56) s'étendant dans l'alésage (28) et relié au collet (24).

10. Dispositif selon l'une quelconque des revendications précédentes, comprenant en outre un moyen de verrouillage (86, 96) pour bloquer de façon amovible une pièce (80, 94) dans l'alésage (66) du collet (64) de manière que la pièce (80, 94) puisse être insérée et remplacée sans retrait du dispositif d'accès percutané (60) d'une position implantée et sans provoquer de perturbations substantielles à la peau et au tissu (74) proches dudit dispositif, et des moyens de verrouillage (86, 96) comprenant des moyens (90) pour éviter la torsion dudit collet (64) pendant le verrouillage et le dégagement de ladite pièce (80, 94).

11. Dispositif selon la revendication 10, dans lequel la pièce comprend un tube (76) pour acheminer une substance, et ledit moyen pour bloquer de façon amovible le tube comprend un joint flexible (80) conformé de manière à pouvoir se disposer dans l'alésage (70) dudit collet (64) et entourer une partie dudit tube, et un capuchon (86) comprenant un trou traversant pour loger le tube, ledit capuchon (86) pouvant être fixé au collet (64) et étant adapté à comprimer le joint (80) contre le tube (76) et le collet (64).

12. Dispositif selon la revendication 10, dans lequel la pièce comprend un bouchon (94) conformé en vue de son insertion dans l'alésage (66) du collet (64) et le moyen Pour verrouiller de façon amovible le bouchon (94) comprend un capuchon (96) pouvant être fixé au collet (64) par dessus le bouchon (94).

13. Dispositif selon la revendication 11, dans lequel le capuchon comprend un anneau fileté pouvant coulisser le long du tube, et le collet (64) comprend une partie filetée (84) pouvant coopérer avec le capuchon.

14. Dispositif selon l'une quelconque des revendications 10 à 13, dans lequel les moyens pour éviter la torsion comprennent une paire d'évidements (90) dans la surface extérieure du collet (64) Pour permettre le maintien du collet (64) en position pendant le verrouillage et le dégagement de la pièce (80, 94).

15. Dispositif selon la revendication 11, dans lequel une partie du collet (64) est conformée pour former une cavité conique (78) dans l'alésage (66) et le joint flexible (80) comprend un matériau élastomère pouvant être logé dans la cavité (78).

20

40

26

26

2

56

58

36

34

24

28

22

2

# Fig. 1.

20

56

33

58

32

34

28

26

40

36

35

22

26

58

41

# Fig. 2.

1

Fig. 3.

Fig. 4.

Fig. 5.